# EUROPEAN PATENT SPECIFICATION

(11) **EP 2 895 469 B1**
(45) Date of publication and mention of the grant of the patent: **18.10.2017**
(21) Application number: 13759215.0
(22) Date of filing: 09.09.2013
(51) Int. Cl.: C07D 263/22, A61K 31/421, A61P 3/10

(54) **SOLID FORMS OF (S)-2-METHOXY-3-{4-[2-(5-METHYL-2-PHENYL-OXAZOL-4-YL)-ETHOXY]-BENZO[B]THIOPHEN-7-YL}-PROPIONIC ACID AND OF SALTS THEREOF**
FESTE FORMEN VON (S)-2-METHOXY-3-{4-[2-(5-METHYL-2-PHENYL-OXAZOL-4-YL)-ETHOXY]-BENZO[B]THIOPHEN-7-YL}-PROPIONIC SAUER UND SALZE DAVON
FORMES SOLIDES DE L'ACIDE (S)-2-METHOXY-3-{4-[2-(5-METHYL-2-PHENYL-OXAZOL-4-YL)-ETHOXY]-BENZO[B]THIOPHEN-7-YL}-PROPIONIQUE ET DE SES SELS

(30) Priority: 12.09.2012 EP 12184009
(43) Date of publication of application: 22.07.2015
(73) Proprietor: F. Hoffmann-La Roche AG, 4070 Basel (CH)
(72) Inventor: DREIER, Lukas, CH-4058 Basel (CH); EGLI, André, CH-4053 Basel (CH); FAEHNRICH, Karsten, 79639 Grenzach-Wyhlen (DE); GRASSMANN, Olaf, 79540 Loerrach (DE); MOHR, Peter, CH-4054 Basel (CH); SCHWITTER, Urs, CH-4153 Reinach BL (CH); STAHR, Helmut, 79539 Loerrach (DE); WYTTENBACH, Nicole, CH-4450 Sissach (CH)
(74) Representative: Pomeranc, Didier
(86) International application number: PCT/EP2013/068561
(87) International publication number: WO 2014/040936

(56) References cited:
- WO-A1-02/092084
- WO-A1-2010/108861

## Description

The invention relates in particular to solid Form A of sodium (S)-2-methoxy-3-{4-[2-(5-methyl-2-phenyl-oxazol-4-yl)-ethoxy]-benzo[b]thiophen-7-yl}-propanoate.

(S)-2-methoxy-3-{4-[2-(5-methyl-2-phenyl-oxazol-4-yl)-ethoxy]-benzo[b]thiophen-7-yl}-propionic acid has been described e.g. in WO 02/092084. It is particularly useful, on top of background therapy for other risk factors, for the reduction of cardiovascular mortality, non-fatal myocardial infarction and stroke, in adults with type 2 diabetes following a recent acute coronary syndrome. It is particularly useful in the treatment or prevention of type 2 diabetes, insulin resistance, acute coronary syndrome, diabetic nephropathy or diabetic retinopathy.

The invention therefore also relates to a solid form according to the invention for the the reduction of cardiovascular mortality, non-fatal myocardial infarction and stroke, in adults with type 2 diabetes following a recent acute coronary syndrome, or for the treatment or prevention of type 2 diabetes, insulin resistance, acute coronary syndrome, diabetic nephropathy or diabetic retinopathy.

It is now described that several solid forms of (S)-2-methoxy-3-{4-[2-(5-methyl-2-phenyl-oxazol-4-yl)-ethoxy]-benzo[b]thiophen-7-yl}-propionic acid and its salts can be prepared.

As used herein, "solid forms" denotes a material in the solid state as understood by the skilled person, and comprises in particular the amorphous solid state and the crystalline solid state.

As used herein, "amorphous form" denotes a material that lacks long range order and as such does not show sharp X-ray diffraction peaks in its XRPD pattern. The XRPD pattern of an amorphous material is characterized by one or more amorphous halos.

An "amorphous halo" is an approximately bell-shaped diffraction maximum in the X-ray powder diffraction pattern of an amorphous substance. The FWHM (full width at half maximum) of an amorphous halo is usually bigger than two degrees in 2Theta.

"API" is used herein as an acronym of active pharmaceutical ingredient.

"DSC" is used herein as an acronym of Differential Scanning Calorimetry.

"DVS" is used herein as an acronym of Dynamic Vapor Sorption.

"RH" is used herein as an acronym of relative humidity.

When used alone, "Free acid Form A" is used herein as an abbreviation for the crystalline form A of (S)-2-methoxy-3-{4-[2-(5-methyl-2-phenyl-oxazol-4-yl)-ethoxy]-benzo[b]thiophen-7-yl}-propionic acid.

When used alone, "Free acid Form B" is used herein as an abbreviation for the crystalline form B of (S)-2-methoxy-3-{4-[2-(5-methyl-2-phenyl-oxazol-4-yl)-ethoxy]-benzo[b]thiophen-7-yl }-propionic acid.

When used alone, "Free acid Form C" is used herein as an abbreviation for the crystalline form C of (S)-2-methoxy-3-{4-[2-(5-methyl-2-phenyl-oxazol-4-yl)-ethoxy]-benzo[b]thiophen-7-yl} -propionic acid.

When used alone, "Free acid Form D" is used herein as an abbreviation for the crystalline form D of (S)-2-methoxy-3-{4-[2-(5-methyl-2-phenyl-oxazol-4-yl)-ethoxy]-benzo[b]thiophen-7-yl }-propionic acid.

When used alone, "Free acid Form E" is used herein as an abbreviation for the crystalline form E of (S)-2-methoxy-3-{4-[2-(5-methyl-2-phenyl-oxazol-4-yl)-ethoxy]-benzo[b]thiophen-7-yl}-propionic acid.

When used alone, "Sodium salt Form A" is used herein as an abbreviation for the crystalline form A of sodium (S)-2-methoxy-3-{4-[2-(5-methyl-2-phenyl-oxazol-4-yl)-ethoxy]-benzo[b]thiophen-7-yl}-propanoate.

When used alone, "Sodium salt Form B" is used herein as an abbreviation for the crystalline form B of sodium (S)-2-methoxy-3-{4-[2-(5-methyl-2-phenyl-oxazol-4-yl)-ethoxy]-benzo[b]thiophen-7-yl -propanoate.

When used alone, "Sodium salt Form C" is used herein as an abbreviation for the crystalline form C of sodium (S)-2-methoxy-3-{4-[2-(5-methyl-2-phenyl-oxazol-4-yl)-ethoxy]-benzo[b]thiophen-7-yl -propanoate.

When used alone, "Diisopropylammonium salt Form A" is used herein as an abbreviation for the crystalline form A of diisopropylammonium (S)-2-methoxy-3-{4-[2-(5-methyl-2-phenyl-oxazol-4-yl)-ethoxy]-benzo[b]thiophen-7-yl}-propanoate.

"Free acid" is used herein as the abbreviation of (S)-2-methoxy-3-{4-[2-(5-methyl-2-phenyl-oxazol-4-yl)-ethoxy]-benzo[b]thiophen-7-yl}-propionic acid.

"Sodium salt" is used herein as the abbreviation of the sodium salt of (S)-2-methoxy-3-{4-[2-(5-methyl-2-phenyl-oxazol-4-yl)-ethoxy]-benzo[b]thiophen-7-yl}-propionic acid.

"Diisopropylammonium salt" is used herein as the abbreviation of the diisopropylammonium salt of (S)-2-methoxy-3-{4-[2-(5-methyl-2-phenyl-oxazol-4-yl)-ethoxy]-benzo[b]thiophen-7-yl}-propionic acid.

"IR" is used herein as an acronym of InfraRed spectroscopy.

"Raman" is used herein as an acronym of Raman spectroscopy.

"XRPD" is used herein as an acronym of X-Ray Powder Diffraction.

"TGA" is used herein as an acronym of ThermoGravimetric Analysis.

DSC curves were recorded using a Mettler-Toledo™ differential scanning calorimeter DSC820, DSC821 or DSC1 with a FRS05 sensor. System suitability tests were performed with indium as reference substance and calibrations were carried out using indium, benzoic acid, biphenyl and zinc as reference substances.

For the measurements, approximately 2 - 6 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 100 mL/min using heating rates of usually 10 K/min.

TGA analysis was performed on a Mettler-Toledo™ thermogravimetric analyzer (TGA850 or TGA851). System suitability tests were performed with Hydranal as reference substance and calibrations using aluminum and indium as reference substances.

For the thermogravimetric analyses, approx. 5 to 10 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 50 mL/min using a heating rate of 5 K/min.

DVS isotherms were collected on a DVS-1, DVS-HT and DVS Intrinsic (SMS Surface Measurements Systems) moisture balance system. The sorption/desorption isotherms were measured stepwise in a range of 0% RH to 90% RH at 25 °C. A weight change of <0.002 mg/min was chosen as criterion to switch to the next level of relative humidity (with a maximum equilibration time of 24 hours, if the weight criterion was not met). The data were corrected for the initial moisture content of the samples; that is, the weight after drying the sample at 0% RH was taken as the reference point.

IR spectra were recorded as film of a Nujol suspension of approximately 5 mg of sample and few Nujol between two sodium chloride plates, with an FTIR spectrometer in transmittance. The spectrometer is a Nicolet™ Magna 860 (Thermo) or equivalent (resolution 2 cm-1, 100 or more coadded scans, DTGS detector).

Raman spectra were recorded with a dispersive Raman spectrometer (ARAMIS, HoribaJY) using the 180-degree reflective configuration of a microscope with an x50 objective (numerical aperture 0.75). The instrument is equipped with a He-Ne Laser (emits at 633 nm), a grating of 1200 lines/mm, resulting in a spectral resolution of approximately 5 cm-1. For detection a CCD is used. Time and number of exposures to the excitations was adjusted to the Raman cross section of the sample (range 3-5 times 3-7s).

X-ray powder diffraction patterns were recorded at ambient conditions in transmission geometry with a STOE STADI P diffractometer (Cu Kα1 radiation, primary monochromator, position sensitive detector, angular range 3° to 42° 2Theta, approximately 60 minutes total measurement time). Approximately 25 mg of sample were prepared and analyzed without further processing (e.g. grinding or sieving) of the substance.

The invention thus relates in particular to Sodium salt Form A and to its preparation process as described below.

In the present description, temperatures are given with an uncertainty in the measurement of ± 2 °C. In this context, "ca." means approximately and therefore means ± 2 °C.

Free acid Form A is the thermodynamically most stable polymorph of (S)-2-methoxy-3-{4-[2-(5-methyl-2-phenyl-oxazol-4-yl)-ethoxy]-benzo[b]thiophen-7-yl}-propionic acid below ca. 60 °C. It is non-hygroscopic. Free acid Form A may be formed upon spontaneous or seeded solution mediated phase transformation or upon spontaneous or seeded crystallization from a variety of organic solvents or thermally by incubation of Free acid Form B at low temperature.

Free acid Form A transforms into Free acid Form B at a temperature superior to 85 °C (temperature controlled XRPD). Spontaneous solid-solid retransformation of Free acid Form B into Free acid Form A is observed at ca. 45 °C or below. Free acid Form A can be prepared according to the following procedures.

By long-term slurry equilibration: Free acid Form A can be formed by slurry equilibration of any other known solid form (e.g. Free acid amorphous form, Free acid Form B or Free acid Form C) in organic solvents, in particular ethanol, ethanol/water, methanol, isopropanol, ethyl acetate, acetonitrile, acetonitrile/water or isopropyl acetate, more particularly isopropyl acetate. The equilibration can be conducted for multiple days, more particularly for 7 to 30 days, more particularly for 10 to 14 days. It is preferred that the equilibration is conducted at a tempereature not exceeding ca. 60 °C, in particular between 5 °C and 50 °C, more particularly between 25 °C and 30 °C.

By cooling crystallization: Free acid Form A can be formed by cooling crystallization of (S)-2-methoxy-3-{4-[2-(5-methyl-2-phenyl-oxazol-4-yl)-ethoxy]-benzo[b]thiophen-7-yl}-propionic acid from organic solvents, in particular acetone, methyl ethyl ketone or isopropyl acetate, more particularly from isopropyl acetate. It is preferably cooled from a temperature between 40 and 89 °C, more particularly between 75 °C and 85 °C, to a temperature between -20 °C and 20 °C, more particularly between -10 °C and -5 °C. It is in particular cooled down within 2 to 6 hours, more particularly within 2 to 3 hours.

By evaporative crystallization: Free acid Form A can be formed by evaporative crystallization of (S)-2-methoxy-3-{4-[2-(5-methyl-2-phenyl-oxazol-4-yl)-ethoxy]-benzo[b]thiophen-7-yl}-propionic acid from organic solvents, in particular from ethanol, ethanol/water, methanol, methyl ethyl ketone, acetone, ethyl acetate, t-butyl methyl ether, dioxane, tetrahydrofuran, 2-methyl tetrahydrofuran, toluene or dimethyl carbonate, more particularly from ethanol. The evaporative crystallization is conducted at a temperature not exceeding ca. 60 °C, in particular at ambient temperature or at ca. 22 °C.

A particularly advantageaous process for the manufacture of the solid form A of (S)-2-methoxy-3-{4-[2-(5-methyl-2-phenyl-oxazol-4-yl)-ethoxy]-benzo[b]thiophen-7-yl}-propionic acid comprises the following steps:
(a) Dissolving (S)-2-methoxy-3-{4-[2-(5-methyl-2-phenyl-oxazol-4-yl)-ethoxy]-benzo[b]thiophen-7-yl}-propionic acid in an organic solvent;
(b) Heating the solution in order to obtain a clear solution of (S)-2-methoxy-3-{4-[2-(5-methyl-2-phenyl-oxazol-4-yl)-ethoxy]-benzo[b]thiophen-7-yl}-propionic acid;
(c) Cooling the solution to a temperature of ca. 60 °C or below; and
(d) Isolating the solid form at the temperature of step (c).

The organic solvent of step (a) is in particular acetone, methyl ethyl ketone or isopropyl acetate, preferably isopropyl acetate.

In step (b), the solution is preferably prepared at reflux conditions, depending on the solvent preferably between 40 °C and 89 °C, for example 40 °C, 56 °C or 89 °C.

In step (c), the solution is particularly preferably cooled down to a temperature inferior or equal to 50 °C. A temperature inferior or equal to -5 °C is more particularly preferred for step (c).

The solid form can be isolated in step (d) in particular by filtration. This step is most preferably carried out at a temperature inferior or equal to ca. 60 °C, in particular inferior or equal to 50 °C, preferably inferior or equal to -5 °C.

The filtration can comprise the rinsing of the isolated solid form with an organic solvent, in particular acetone, methyl ethyl ketone or isopropyl acetate, preferably isopropyl acetate. The organic solvent used for rinsing the isolated solid form is preferably at a temperature inferior or equal to 60 °C, in particular inferior or equal to 50 °C, preferably inferior or equal to -5 °C.

When in step (c) the solution is cooled down to a temperature inferior or equal to -5 °C, the most robust process is achieved. Form A of (S)-2-methoxy-3-{4-[2-(5-methyl-2-phenyl-oxazol-4-yl)-ethoxy]-benzo[b]thiophen-7-yl}-propionic acid is thereby obtained with the highest yield and selectivity.

Step (d) can be followed by a step (e) wherein the polymorph is dried. This drying step has to be carried out at 80 °C or below in order to avoid the formation of Free acid Form B. Step (e) can occur at room temperature. Reduced pressure can also be used. The temperature of step (e) can therefore be for example between room temperature and 80 °C. It is particularly preferred to dry Free acid Form A at a temperature not exceeding ca 60 °C, for example between room temperature and 60 °C.

A further particular process for the manufacture of the solid form A of (S)-2-methoxy-3-{4-[2-(5-methyl-2-phenyl-oxazol-4-yl)-ethoxy]-benzo[b]thiophen-7-yl}-propionic acid comprises the following steps:
(a) Dissolving (S)-2-methoxy-3-{4-[2-(5-methyl-2-phenyl-oxazol-4-yl)-ethoxy]-benzo[b]thiophen-7-yl}-propionic acid in an organic solvent, preferably isopropyl acetate;
(b) Heating the solution to reflux in order to obtain a clear solution of (S)-2-methoxy-3-{4-[2-(5-methyl-2-phenyl-oxazol-4-yl)-ethoxy]-benzo[b]thiophen-7-yl}-propionic acid;
(c) Cooling the solution to a temperature inferior or equal to 50 °C; and
(d) Isolating the solid form at the temperature of step (c), preferably by filtration, and optionally rinsing the isolated solid form with isopropyl acetate, preferably at 50 °C.
(e) Optionaly drying the isolated solid form at a temperature inferior or equal to 80 °C.

A further particular process for the manufacture of the solid form A of (S)-2-methoxy-3-{4-[2-(5-methyl-2-phenyl-oxazol-4-yl)-ethoxy]-benzo[b]thiophen-7-yl}-propionic acid, comprises the following steps:
(a) Dissolving (S)-2-methoxy-3-{4-[2-(5-methyl-2-phenyl-oxazol-4-yl)-ethoxy]-benzo[b]thiophen-7-yl}-propionic acid in isopropyl acetate;
(b) Heating the solution to reflux in order to abtain a clear solution of (S)-2-methoxy-3-{4-[2-(5-methyl-2-phenyl-oxazol-4-yl)-ethoxy]-benzo[b]thiophen-7-yl}-propionic acid;
(c) Cooling the solution to a temperature of -5 °C;
(d) Isolating the solid form at the temperature of step (c) by filtration and optionally rinsing the isolated solid form with isopropyl acetate at -5 °C; and
(e) Optionally drying the isolated solid form at a temperature inferior or equal to 60 °C.

The process described above allows a consistent and convenient manufacture of pure Free acid Form A.

Free acid Form A can be characterized by an X-ray powder diffraction pattern obtained with a Cu Kα radiation having characteristic peaks expressed in degrees 2Theta at approximately: 4.9, 8.1, 11.8, 13.2, 13.9, 14.9, 18.5, 21.0, 21.6, 22.7, 25.3, 26.2 and 28.5. The term "approximately" means in this context that there is an uncertainty in the measurements of the degrees 2Theta of ± 0.2 (expressed in degrees 2Theta).

Free acid Form A can be furher characterized by an infrared spectrum having sharp bands at: 3097, 3078, 3061, 3040, 1745, 1705, 1653, 1605, 1597, 1576, 1555, 1508, 1343, 1315, 1303, 1290, 1272, 1261, 1242, 1231, 1183, 1167, 1153, 1124, 1086, 1073, 1062, 1037, 1022, 936, 803, 760, 716, 694, 688, 667, 655, 615 and 626 cm-1 (± 2 cm-1).

Free acid Form A can be furher characterized by a Raman spectrum having sharp bands at: 3102, 3081, 3054, 2985, 2932, 2919, 1746, 1654, 1607, 1578, 1556, 1509, 1476, 1463, 1454 , 1402, 1357, 1344, 1331, 1262, 1244, 1182, 1088, 1029, 1003, 976, 961, 801, 778, 682, 658, 617, 494, 419, 350, 337, 312, 276, 229, 196 and 178 cm-1 (± 3 cm-1).

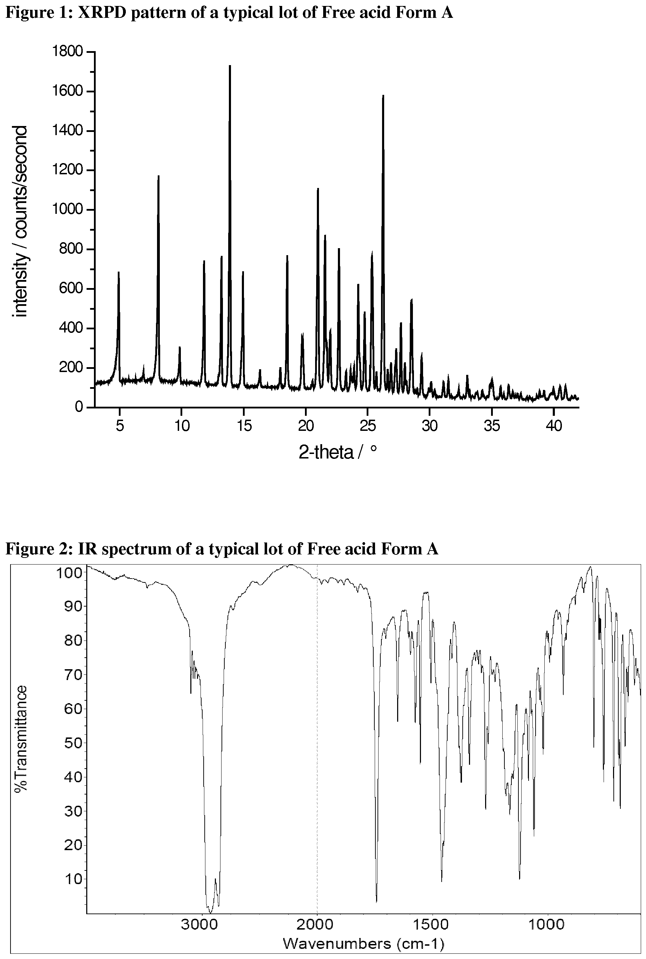

These characteristics and others are shown on figures 1 to 3.

Free acid Form B may be formed upon spontaneous or seeded solution mediated phase transformation, or upon spontaneous or seeded crystallization from a variety of organic solvents, or thermally by incubation of Free acid Form A at elevated temperature. Free acid Form B is non-hygroscopic.

Free acid Form B exhibits a melting temperature of ca. 153 °C (extrapolated peak DSC). At below ca. -5 °C (extrapolated peak DSC) Free acid Form B spontaneously transforms into Free acid Form A.

Free acid Form B can be prepared according to the following procedures.

By long-term slurry equilibration: Free acid Form B can be formed by slurry equilibration of any other known solid Form (e.g. Free acid amorphous form, Free acid Form A or Free acid Form C) in organic solvents, in particular dioxane, toluene or isopropyl acetate, more particularly isopropyl acetate. The long-term slurry equilibration can be conducted in particular for multiple days, particularly for 7 to 14 days. The long-term slurry equilibration is performed at a temperature between 80 °C and 120 °C, more particularly between 90 °C and 100 °C.

By cooling crystallization: Free acid Form B can be formed by cooling crystallization of (S)-2-methoxy-3-{4-[2-(5-methyl-2-phenyl-oxazol-4-yl)-ethoxy]-benzo[b]thiophen-7-yl}-propionic acid from organic solvents, in particular chloroform, from 65 °C to 20 °C, in particular within 2 hours.

By evaporative crystallization: Free acid Form B can be formed by evaporative crystallization of (S)-2-methoxy-3-{4-[2-(5-methyl-2-phenyl-oxazol-4-yl)-ethoxy]-benzo[b]thiophen-7-yl}-propionic acid from organic solvents, in particular chloroform, in particular at ambient temperature or at ca. 25 °C.

By high temperature exposure: Free acid Form B can be formed by incubation of Free acid Form A between 80 °C and the melting temperature of Form B, particularly between 80 °C and 150 °C, more particularly between 85 °C and 145 °C, more particularly between 100 °C and 120 °C. Free acid Form B can also be formed by incubation of the amorphous form between 60 °C and 145 °C, more particularly between 80 °C and 120 °C.

Free acid Form B can be characterized by an X-ray powder diffraction pattern obtained with a Cu Kα radiation having characteristic peaks expressed in degrees 2Theta at approximately: 5.0, 8.1, 12.0, 13.0, 13.7, 14.9, 18.9, 21.3, 24.2 and 26.3. The term "approximately" means in this context that there is an uncertainty in the measurements of the degrees 2Theta of ± 0.2 (expressed in degrees 2Theta).

Free acid Form B can be also characterized by an infrared spectrum having sharp bands at 3094, 3077, 3059, 1744, 1653, 1605, 1596, 1575, 1555, 1508, 1418, 1342, 1303, 1272, 1261, 1233, 1196, 1183, 1167, 1153, 1124, 1086, 1062, 1036, 1022, 761, 717, 707, 693, 688, 667, 655 and 626 cm-1 (± 2 cm-1).

Free acid Form B can be also characterized by a Raman spectrum having sharp bands at 3099, 3081, 2923, 2886, 1658, 1610, 1580, 1560, 1511, 1494, 1478, 1466, 1457, 1404, 1391, 1362, 1346, 1333, 1266, 1245, 1182, 1092, 1031, 1005, 977, 963, 805, 780, 684, 662, 620, 546, 497, 465, 452, 436, 422, 383, 352, 335, 314, 278, 232 and 183 cm-1 (± 3 cm-1).

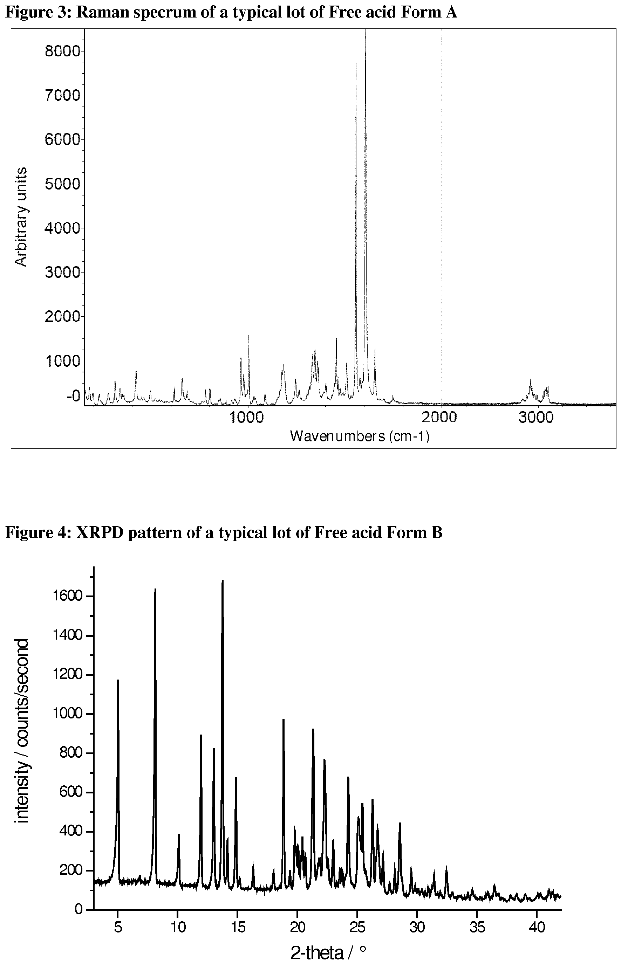

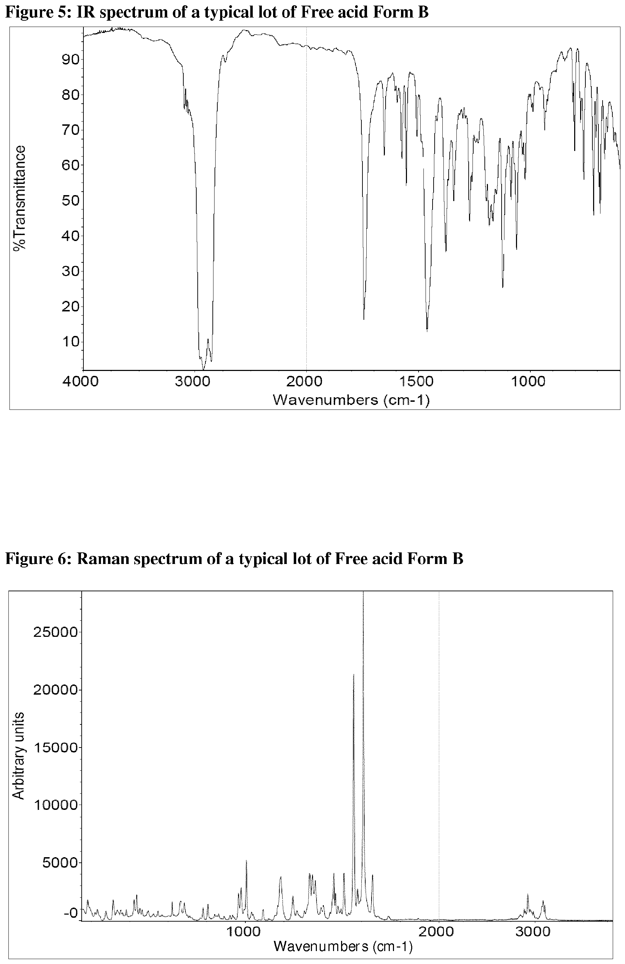

These characteristics and others are shown on figures 4 to 6.

Polymorphic Free acid Form C may be formed upon spontaneous or seeded solution mediated phase transformation or upon spontaneous or seeded crystallization from a variety of organic solvents. At ambient temperature the non-hygroscopic Free acid Form C is thermodynamically less stable than Free acid Form A and Free acid Form B. Free acid Form C transforms into Free acid Form B at ca. 150 °C (extrapolated peak DSC).

Free acid Form C can be prepared according to the following procedures.

By cooling crystallization: Free acid Form C can be formed by cooling crystallization of (S)-2-methoxy-3-{4-[2-(5-methyl-2-phenyl-oxazol-4-yl)-ethoxy]-benzo[b]thiophen-7-yl}-propionic acid from 65 to 5 °C and from organic solvents, in particular from trifluoroethanol. The cooling crystallization can be carried out within 8 h.

By evaporative crystallization: Free acid Form C can be formed by evaporative crystallization of (S)-2-methoxy-3-{4-[2-(5-methyl-2-phenyl-oxazol-4-yl)-ethoxy]-benzo[b]thiophen-7-yl}-propionic acid from organic solvents, in particular from dichloromethane or isopropanol, in particular at ambient temperature or at ca. 22 °C.

Free acid Form C can be characterized by an X-ray powder diffraction pattern obtained with a Cu Kα radiation having characteristic peaks expressed in degrees 2Theta at approximately: 5.0, 8.0, 8.3, 12.8, 13.6, 14.0, 16.7, 21.0 and 23.0. The term "approximately" means in this context that there is an uncertainty in the measurements of the degrees 2Theta of ± 0.2 (expressed in degrees 2Theta).

Free acid Form C can also be characterized by an infrared spectrum having sharp bands at 3093, 3078, 3044, 1738, 1653, 1595, 1576, 1554, 1507, 1450, 1434, 1419, 1384, 1341, 1332, 1312, 1299, 1288, 1273, 1249, 1198, 1184, 1126, 1119, 1087, 1064, 1037, 1026, 988, 937, 929, 919, 809, 800, 776, 762, 719, 706, 690, 667, 656 and 629 cm-1 (± 2 cm-1).

Free acid Form C can also be characterized by a Raman spectrum having sharp bands at 3098, 3079, 2921, 2885, 1654, 1608, 1578, 1555, 1509, 1476, 1464, 1453, 1403, 1388, 1361, 1343, 1329, 1244, 1179, 1161, 1089, 1027, 1005, 975, 964, 803, 781, 683, 664, 616, 495, 464, 435, 421, 381, 335, 312 and 183 cm-1 (± 3 cm-1).

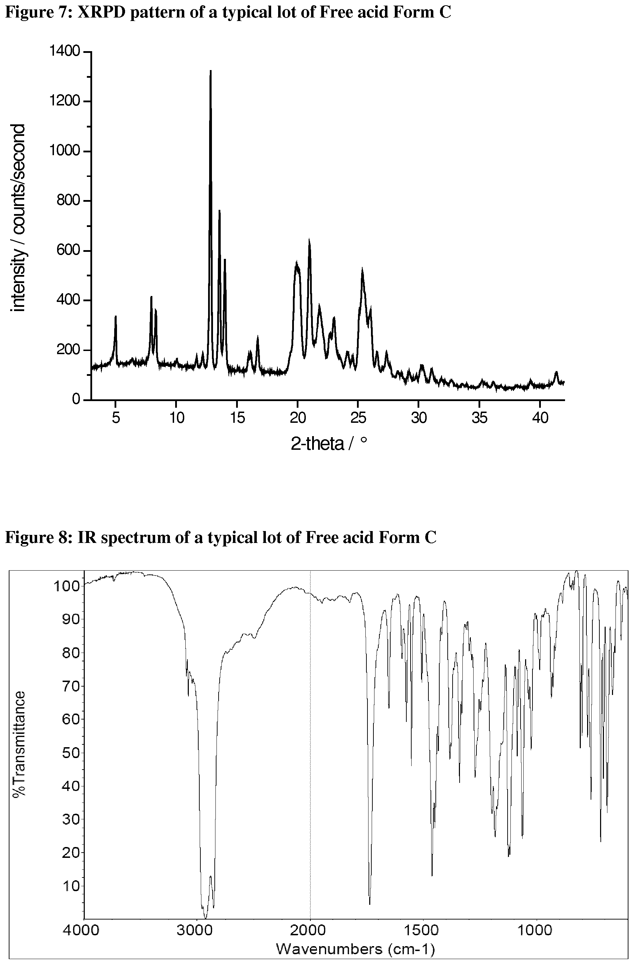

These characteristics and others are shown on figures 7 to 9.

Free acid Form D is a chloroform mono solvate of (S)-2-methoxy-3-{4-[2-(5-methyl-2-phenyl-oxazol-4-yl)-ethoxy]-benzo[b]thiophen-7-yl}-propionic acid. The labile solid form transforms into Free acid Form B upon storage at ambient conditions over time. Free acid Form D may be formed upon spontaneous or seeded crystallization from chloroform.

Free acid Form D can be prepared according to the following procedure:
By cooling crystallization: Free acid Form D can be formed by cooling crystallization of (S)-2-methoxy-3-{4-[2-(5-methyl-2-phenyl-oxazol-4-yl)-ethoxy]-benzo[b]thiophen-7-yl}-propionic acid from chloroform. It is preferably cooled from a temperature between 55 and 65 °C, more particularly from 60 °C, to a temperature between 5 °C and 20 °C, more particularly 10 °C. It is in particular cooled down within 6 to 12 hours, more particularly within 8 hours. The sample must be handled in wet stage to avoid transformation into Free acid Form B.

Free acid Form D can be characterized by an X-ray powder diffraction pattern obtained with a Cu Kα radiation having characteristic peaks expressed in degrees 2Theta at approximately: 4.9, 13.1, 14.8, 16.5, 19.2, 19.7, 20.0, 20.8, 21.8, 22.1, 22.8, 24.5, 24.9, 26.1 and 29.1. The term "approximately" means in this context that there is an uncertainty in the measurements of the degrees 2Theta of ± 0.2 (expressed in degrees 2Theta).

Free acid Form D can be furher characterized by an infrared spectrum having sharp bands at: 1735, 1654, 1593, 1574, 1554, 1506, 1345, 1271, 1238, 1204, 1182, 1145, 1117, 1063, 1027, 990, 938, 928, 879, 850, 800, 762, 717, 707, 693, 687, 663, and 629 cm-1 (± 2 cm-1).

Free acid Form D can be furher characterized by a Raman spectrum having sharp bands at: 3068, 3023, 2927, 1655, 1607, 1576, 1556, 1508, 1452, 1347, 1338, 1261, 1239, 1179, 1087, 1004, 976, 805, 770, 670, 617, 465, 438, 369, 328, and 263 cm-1 (± 3 cm-1).

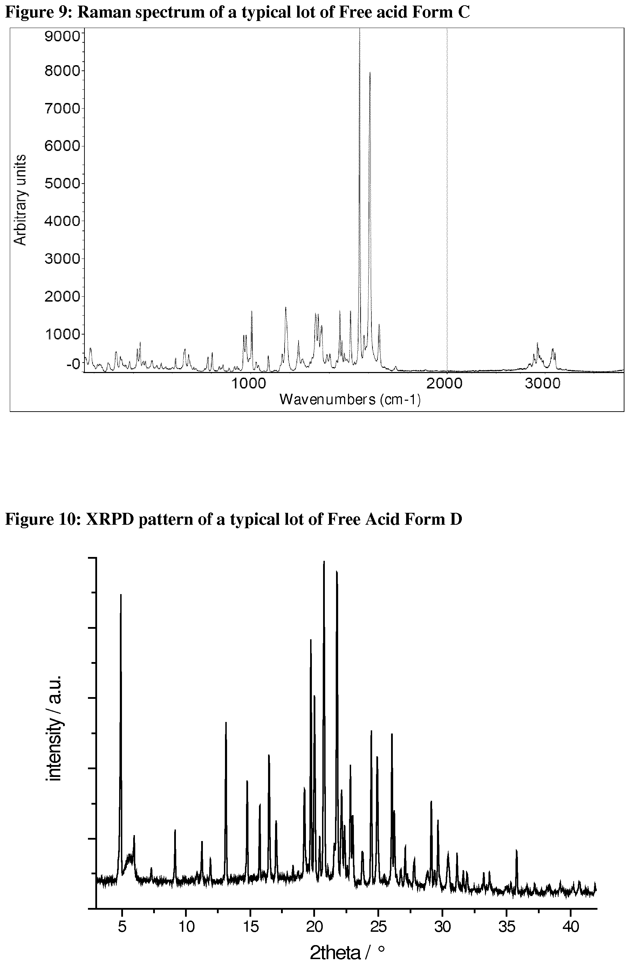

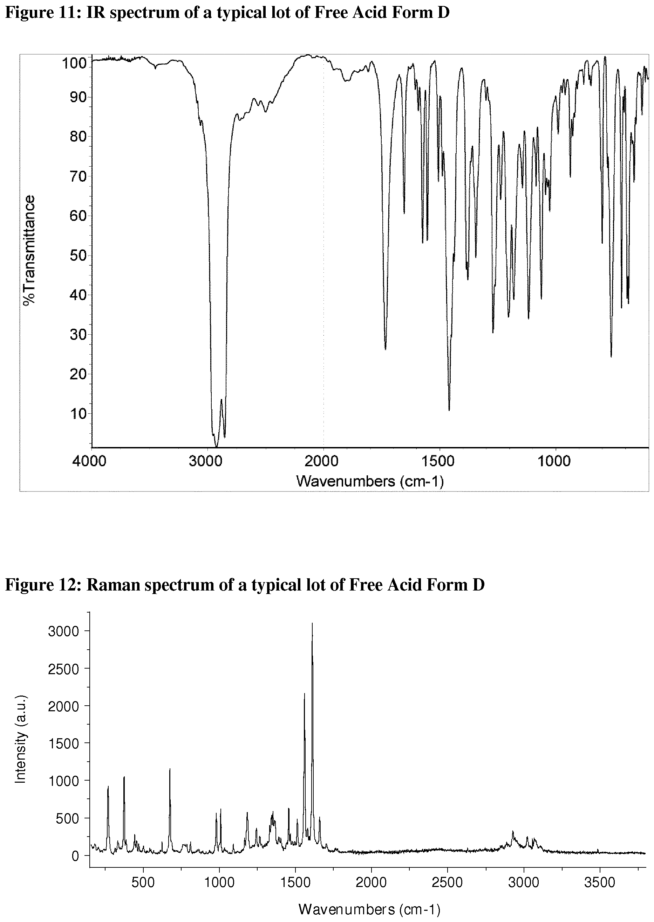

These characteristics and others are shown on figures 10 to 12.

Free acid Form E is a dichloromethane mono solvate of (S)-2-methoxy-3-{4-[2-(5-methyl-2-phenyl-oxazol-4-yl)-ethoxy]-benzo[b]thiophen-7-yl}-propionic acid. The labile solid form transforms into Free acid Form B upon mild heating. Free acid Form E may be formed upon spontaneous or seeded crystallization from dichloromethane.

Free acid Form E can be prepared according to the following procedures:
By cooling crystallization: Free acid Form E can be formed by cooling crystallization of (S)-2-methoxy-3-{4-[2-(5-methyl-2-phenyl-oxazol-4-yl)-ethoxy]-benzo[b]thiophen-7-yl}-propionic acid from dichloromethane. It is preferably cooled from a temperature between 55 and 65 °C, more particularly from 60 °C, to a temperature between 10 °C and 25 °C, more particularly 25 °C. It is in particular cooled down within 6 to 12 hours, more particularly within 8 hours. The sample must be handled in wet stage to avoid transformation into Free acid Form B.

By long-term slurry equilibration: Free acid Form E can be formed by slurry equilibration of any other known solid Form (e.g. Free acid amorphous form, Free acid Form A or Free acid Form C) in dichloromethane. The long-term slurry equilibration can be conducted in particular for multiple days, particularly for 7 to 14 days. The long-term slurry equilibration is performed at a temperature between 15 °C and 25 °C, more particularly at 2 °C. The sample must be handled in wet stage to avoid transformation into Free acid Form B.

Free acid Form E can be characterized by an X-ray powder diffraction pattern obtained with a Cu Kα radiation having characteristic peaks expressed in degrees 2Theta at approximately: 5.0, 5.9, 8.2, 9.2, 11.8, 13.1, 13.8, 14.9, 16.3, 19.9, 20.6, 21.6, 22.0, 22.6, 24.7 and 26.0. The term "approximately" means in this context that there is an uncertainty in the measurements of the degrees 2Theta of ± 0.2 (expressed in degrees 2Theta).

Free acid Form E can be furher characterized by an infrared spectrum having sharp bands at: 3096, 3077, 3061, 1745, 1653, 1575, 1555, 1507, 1343, 1303, 1271, 1262, 1239, 1198, 1183, 1125, 1086, 1062, 1036, 1023, 988, 936, 803, 777, 760, 744, 717, 707, 694, 688, 668, 655, and 627 cm-1 (± 2 cm-1).

Free acid Form E can be furher characterized by a Raman spectrum having sharp bands at: 3068, 2926, 1654, 1607, 1574, 1555, 1507, 1450, 1347, 1336, 1271, 1259, 1237, 1177, 1085, 1002, 973, 803, 704, 662, 615, 435, 378, 324, and 285 cm-1 (± 3 cm-1).

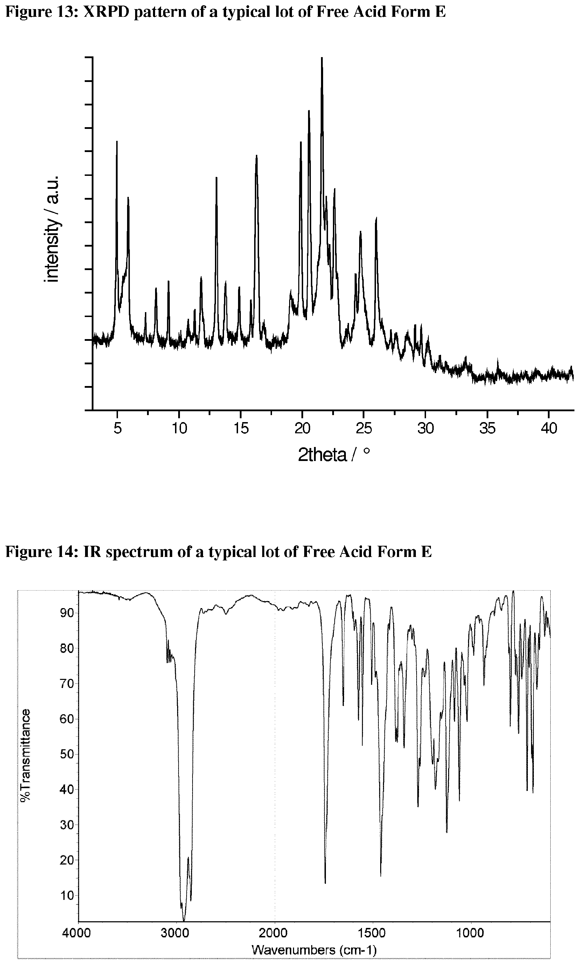

These characteristics and others are shown on figures 13 to 15.

The amorphous form of (S)-2-methoxy-3-{4-[2-(5-methyl-2-phenyl-oxazol-4-yl)-ethoxy]-benzo[b]thiophen-7-yl}-propionic acid may be prepared by various methods. Above the glass transition temperature (approx. 44 °C, midpoint) recrystallization of the hygroscopic amorphous form is likely to occur.

The amorphous form of (S)-2-methoxy-3-{4-[2-(5-methyl-2-phenyl-oxazol-4-yl)-ethoxy]-benzo[b]thiophen-7-yl}-propionic acid may be prepared by the following procedure.

From melt: The amorphous form of the free acid can be prepared from any other known solid form (e.g. Free acid Form A, Free acid Form B or Free acid Form C) by cooling of a melt prepared at >150 °C, more particularly prepared at >180 °C, to ambient temperature.

The amorphous form of the Free acid can be characterized by the lack of sharp X-ray diffraction peaks in its XRPD pattern.

The amorphous form of the Free acid can also be characterized by an infrared spectrum having sharp bands at 3093, 3077, 2728, 1737, 1653, 1595, 1575, 1554, 1507, 1341, 1299, 1272, 1236, 1197, 1185, 1150, 1125, 1087, 1063, 1036, 1025, 988, 959, 937, 929, 809, 800, 776, 762, 718, 706, 689, 665, 656, 628 and 615 cm-1 (± 2 cm-1).

The amorphous form of the Free acid can also be characterized by a Raman spectrum having sharp bands at 3073, 2927, 1648, 1608, 1576, 1556, 1511, 1453, 1399, 1352, 1326, 1237, 1176, 1003, 975, 951, 682, 618 and 438 cm-1 (± 3 cm-1).

The amorphous form of the Free acid can also be characterized by a glass transition temperature (DSC) in the range of approximately 42 °C to 44 °C. The glass transition temperature is largely dependant on the water/solvent content.

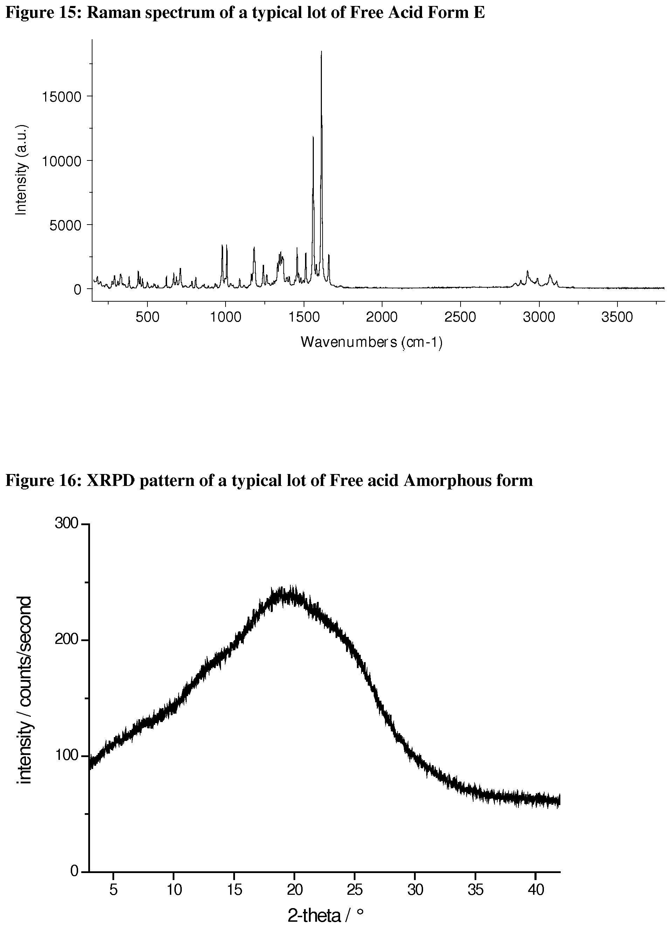

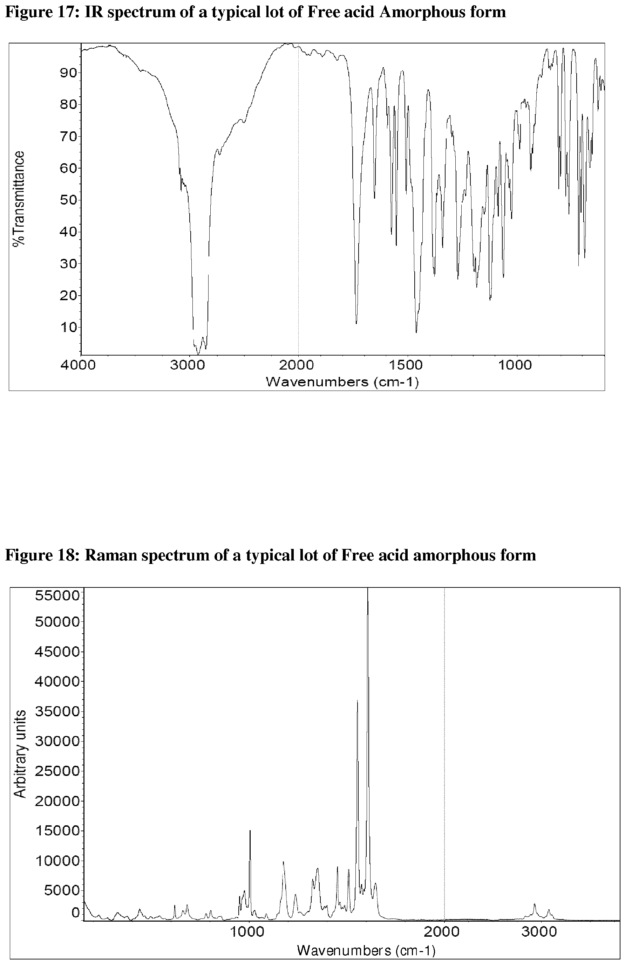

These characteristics and others are shown on figures 16 to 18.

Sodium salt Form A is a polymorphic form of sodium (S)-2-methoxy-3-{4-[2-(5-methyl-2-phenyl-oxazol-4-yl)-ethoxy]-benzo[b]thiophen-7-yl}-propanoate and may be formed upon spontaneous or seeded solution mediated phase transformation, or upon spontaneous or seeded crystallization from a variety of organic solvents, or thermally by incubation of the Sodium salt amorphous form at high temperature (at >115 °C) or of Sodium salt Form B (at >120 °C). Sodium salt Form A transforms into Sodium salt Form B at ca. >208 °C and into a hydrated form at >80% RH (at 25 °C).

Form A of sodium (S)-2-methoxy-3-{4-[2-(5-methyl-2-phenyl-oxazol-4-yl)-ethoxy]-benzo[b]thiophen-7-yl}-propanoate can be prepared according to the following procedures.

By long-term slurry equilibration: Sodium salt Form A can be formed by slurry equilibration of the partially or completely amorphous form or of sodium (S)-2-methoxy-3-{4-[2-(5-methyl-2-phenyl-oxazol-4-yl)-ethoxy]-benzo[b]thiophen-7-yl}-propanoate in organic solvents, particular ethanol/water, between 20 °C and 80 °C, more particularly between 60 °C and 70 °C. The equilibration can be conducted for multiple days, in particularly for 7 to 30 days, more particularly for 14 to 20 days.

By high temperature exposure: Sodium salt Form A can be formed by incubation of the partially or completely amorphous form of sodium (S)-2-methoxy-3-{4-[2-(5-methyl-2-phenyl-oxazol-4-yl)-ethoxy]-benzo[b]thiophen-7-yl}-propanoate between 115 °C and 200 °C, more particularly between 170 °C and 190 °C, more particularly at 180 °C, in particular for 1 to 6 hours, more particularly for 1 to 2 hours.

By cooling crystallization: Sodium salt Form A can be formed by cooling crystallization of sodium (S)-2-methoxy-3-{4-[2-(5-methyl-2-phenyl-oxazol-4-yl)-ethoxy]-benzo[b]thiophen-7-yl}-propanoate from organic solvents, in particular ethanol/water, from a temperature between 50 and 70 °C, more particularly between 60 °C and 65 °C, to a temperature between 0 °C and 20 °C, more particularly between 5 °C and 10 °C, in particular within 6 to 24 hours, more particularly within 12 to 15 hours.

Sodium salt Form A can be characterized by an X-ray powder diffraction pattern obtained with a Cu Kα radiation having characteristic peaks expressed in degrees 2Theta at approximately: 4.7, 4.8, 5.3, 7.9, 8.8, 13.1, 13.5, 13.8, 14.1, 14.5, 17.6, 19.3, 19.9, 21.6, 23.1 and 23.4. The term "approximately" means in this context that there is an uncertainty in the measurements of the degrees 2Theta of ± 0.2 (expressed in degrees 2Theta).

Sodium salt Form A can also be characterized by an infrared spectrum having sharp bands at: 3421, 1617, 1575, 1548, 1508, 1411, 1350, 1338, 1325, 1302, 1260, 1230, 1201, 1176, 1148, 1096, 1066, 1042, 1027, 797, 776, 720 and 689 cm-1 (± 2 cm-1).

Sodium salt Form A can also be characterized by a Raman spectrum having sharp bands at: 3109, 3060, 2927, 1636, 1605, 1580, 1551, 1512, 1491, 1466, 1449, 1387, 1353, 1328, 1307, 1237, 1189, 1176, 1089, 1069, 1019, 1004, 974, 954, 871, 802, 783, 685 , 658, 621, 528, 456, 386 and 345 cm-1 (± 3 cm-1).

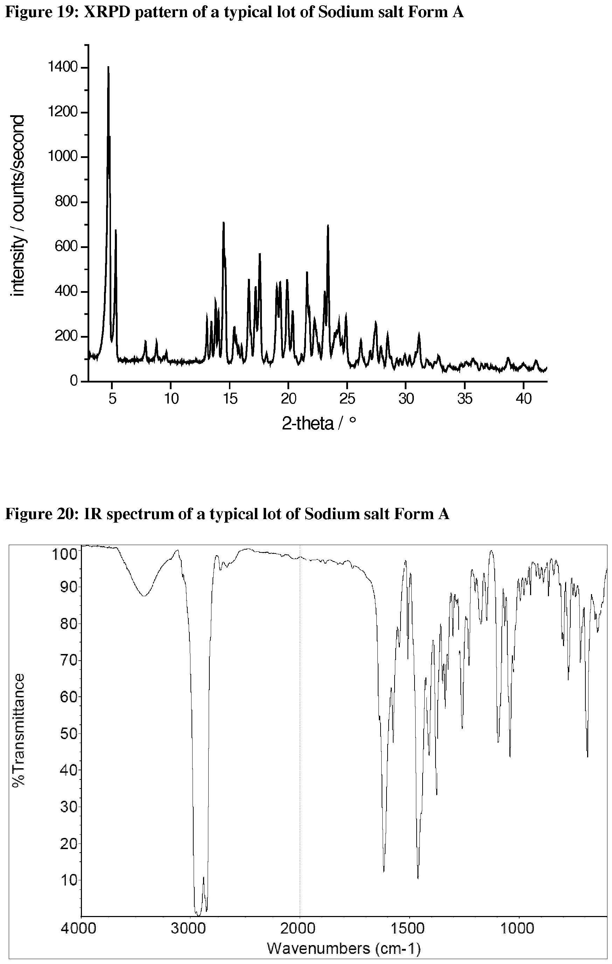

These characteristics and others are shown on figures 19 to 21.

Solid Form B of sodium (S)-2-methoxy-3-{4-[2-(5-methyl-2-phenyl-oxazol-4-yl)-ethoxy]-benzo[b]thiophen-7-yl}-propanoate is metastable and may be formed by incubation of other forms at high temperature.

Sodium salt Form B of sodium (S)-2-methoxy-3-{4-[2-(5-methyl-2-phenyl-oxazol-4-yl)-ethoxy]-benzo[b]thiophen-7-yl}-propanoate can be prepared according to the following procedures.

By high temperature exposure: Sodium salt Form B can be formed by incubation of any other known solid form of sodium (S)-2-methoxy-3-{4-[2-(5-methyl-2-phenyl-oxazol-4-yl)-ethoxy]-benzo[b]thiophen-7-yl}-propanoate (e.g. Sodium salt amorphous form, Sodium salt Form A or Sodium salt hydrated forms) between 210 °C and 240 °C, more particularly between 220 °C and 230 °C, more particularly at 225 °C, particularly for 10 to 20 minutes, more particularly for 15 minutes.

Sodium salt Form B can be characterized by an X-ray powder diffraction pattern obtained with a Cu Kα radiation having characteristic peaks expressed in degrees 2Theta at approximately: 4.7, 8.2 and 9.5. The term "approximately" means in this context that there is an uncertainty in the measurements of the degrees 2Theta of ± 0.2 (expressed in degrees 2Theta).

Sodium salt Form B can also be characterized by an infrared spectrum having sharp bands at: 3058, 1611, 1574, 1554, 1508, 1486, 1410, 1338, 1268, 1234, 1179, 1142, 1102, 1068, 1043, 1024, 949, 931, 908, 846, 796, 774, 715, 692, 664, 625 and 614 cm-1 (± 2 cm-1).

Sodium salt Form B can also be characterized by a Raman spectrum having sharp bands at 1635, 1605, 1550, 1510, 1448, 1409, 1385, 1352, 1327, 1235, 1188, 1175, 1162, 1089, 1017, 1003, 974, 952, 798, 684, 620, 526, 454, 344, 262, 228 and 173 cm-1 (± 3 cm-1).

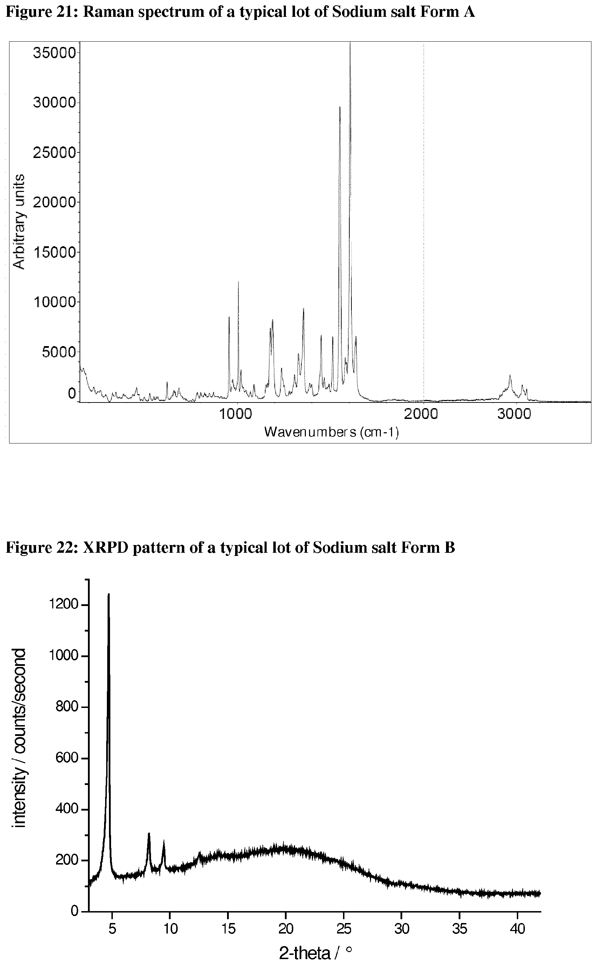

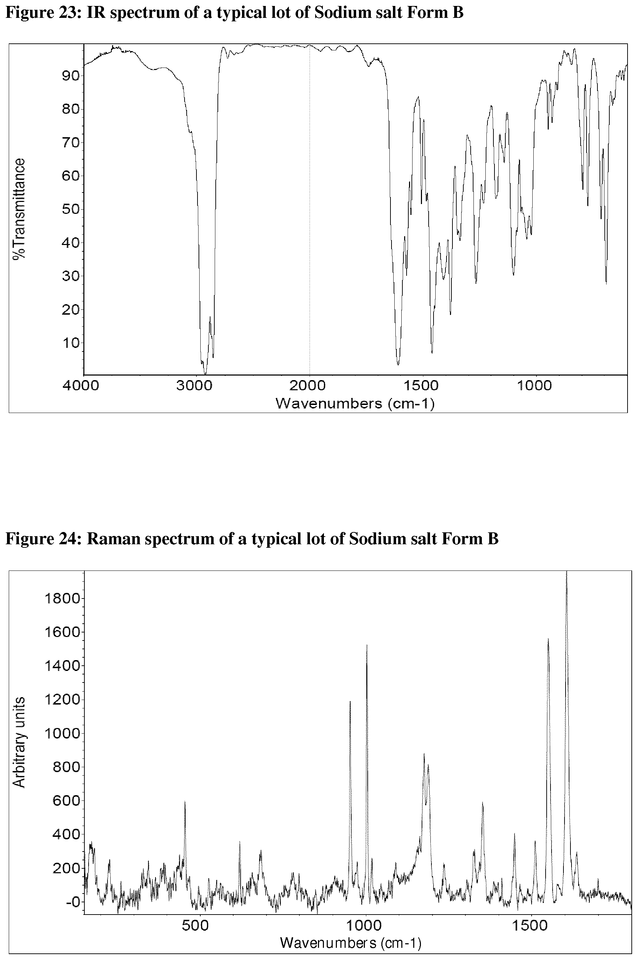

These characteristics and others are shown on figures 22 to 24.

Sodium salt Form C is a hydrated form of sodium (S)-2-methoxy-3-{4-[2-(5-methyl-2-phenyl-oxazol-4-yl)-ethoxy]-benzo[b]thiophen-7-yl}-propanoate and may be formed upon spontaneous or seeded solution mediated phase transformation in aqueous systems or by incubation of other sodium salt forms (e.g. Sodium salt amorphous form, Sodium salt Form A or Sodium Salt *dehydrated Form*) with appropriate atmospheric water activity. At low relative humidity (<15 %-RH at 25 °C), Form C reversibly transforms into another dehydrated solid form, as proven by humidity controlled XRPD.

Form C of sodium (S)-2-methoxy-3-{4-[2-(5-methyl-2-phenyl-oxazol-4-yl)-ethoxy]-benzo[b]thiophen-7-yl}-propanoate can be prepared according to the following procedures:
By exposure to high relative humidity: Form C of the sodium salt of (S)-2-methoxy-3-{4-[2-(5-methyl-2-phenyl-oxazol-4-yl)-ethoxy]-benzo[b]thiophen-7-yl}-propanoate can be formed by incubation of the amorphous form of the sodium salt of (S)-2-methoxy-3-{4-[2-(5-methyl-2-phenyl-oxazol-4-yl)-ethoxy]-benzo[b]thiophen-7-yl}-propanoate at elevated relative humidity, preferably at > 50 %-RH (at 25 °C), more particularly at >80 %-RH (at 25 °C) for 1 to 14 days, more particularly for 5 to 8 days.

By exposure to high relative humidity: Form C of the sodium salt of (S)-2-methoxy-3-{4-[2-(5-methyl-2-phenyl-oxazol-4-yl)-ethoxy]-benzo[b]thiophen-7-yl}-propanoate can be formed by incubation of Form A of the sodium salt of (S)-2-methoxy-3-{4-[2-(5-methyl-2-phenyl-oxazol-4-yl)-ethoxy]-benzo[b]thiophen-7-yl}-propanoate at elevated relative humidity, preferably at > 80 %-RH (at 25 °C), more particularly at >85 %-RH (at 25 °C) for 1 to14 days, more particularly for 5 to 8 days.

By evaporative crystallization: Form C of the sodium salt of (S)-2-methoxy-3-{4-[2-(5-methyl-2-phenyl-oxazol-4-yl)-ethoxy]-benzo[b]thiophen-7-yl}-propanoate can be formed by passive evaporation of an aqueous solution the sodium (S)-2-methoxy-3-{4-[2-(5-methyl-2-phenyl-oxazol-4-yl)-ethoxy]-benzo[b]thiophen-7-yl}-propanoate, at ambient conditions.

Sodium salt Form C can be characterized by an X-ray powder diffraction pattern obtained with a Cu Kα radiation having characteristic peaks expressed in degrees 2Theta at approximately: 4.8, 12.0, 12.8, 14.4, 14.9, 15.8, 16.2, 16.5, 16.8, 20.3, 22.6, 24.2, 25.9 and 26.4. The term "approximately" means in this context that there is an uncertainty in the measurements of the degrees 2Theta of ± 0.2 (expressed in degrees 2Theta).

Sodium salt Form C can also be characterized by an infrared spectrum having sharp bands at: 3396, 3101, 3082, 1641, 1597, 1553, 1509, 1486, 1447, 1422, 1381, 1348, 1335, 1251, 1182, 1153, 1143, 1100, 1058, 1023, 985, 949, 932, 917, 846, 809, 799, 775, 769, 756, 715, 691, 658, 625and 615 cm-1 (± 2 cm-1).

Sodium salt Form C can also be characterized by a Raman spectrum having sharp bands at: 3105, 3069, 3039, 2923, 2880, 2840, 1644, 1606, 1587, 1577, 1555, 1510, 1477, 1461, 1449, 1424, 1398, 1384, 1351, 1336, 1325, 1317, 1236, 1171, 1157, 1144, 1087, 1024, 1002, 969, 949, 884, 846, 800, 774, 681, 662, 616, 538, 461, 436, 345, 315, 275, and 225 cm-1 (± 3 cm-1).

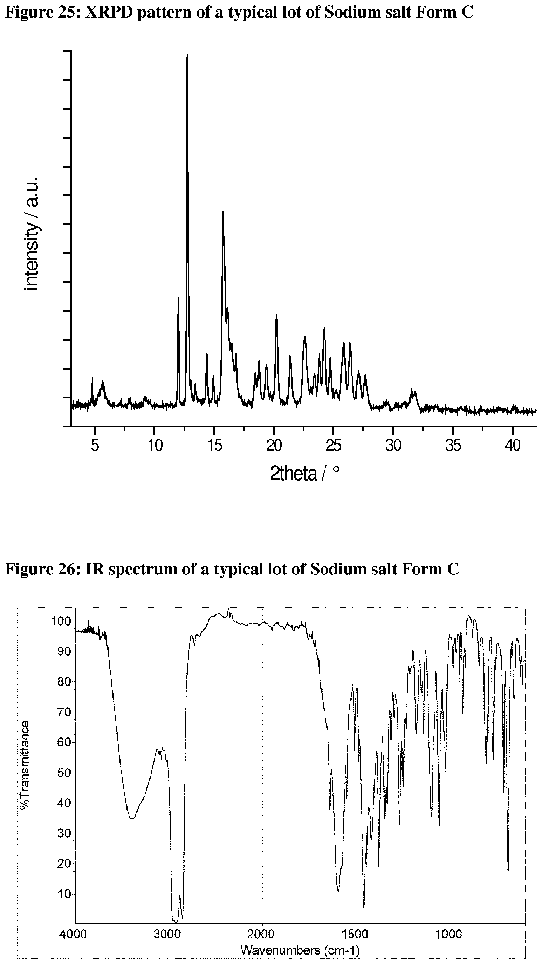

These characteristics and others are shown on figures 25 to 27.

The amorphous form of sodium (S)-2-methoxy-3-{4-[2-(5-methyl-2-phenyl-oxazol-4-yl)-ethoxy]-benzo[b]thiophen-7-yl}-propanoate may be prepared by various methods. Above the glass transition temperature (approx. 65.5 °C, midpoint) recrystallization into Sodium salt Form A of the hygroscopic Sodium salt amorphous form is likely to occur. Above 50% RH (at 25 °C) the Sodium salt amorphous form transforms into hydrated Sodium salt forms.

The amorphous form of sodium (S)-2-methoxy-3-{4-[2-(5-methyl-2-phenyl-oxazol-4-yl)-ethoxy]-benzo[b]thiophen-7-yl}-propanoate can be prepared according to the following procedures.

Freeze-drying: The Sodium salt amorphous form can be prepared by freeze-drying of aqueous solutions of sodium (S)-2-methoxy-3-{4-[2-(5-methyl-2-phenyl-oxazol-4-yl)-ethoxy]-benzo[b]thiophen-7-yl}-propanoate.

By evaporative crystallization: The Sodium salt amorphous form can be prepared by evaporative process of sodium (S)-2-methoxy-3-{4-[2-(5-methyl-2-phenyl-oxazol-4-yl)-ethoxy]-benzo[b]thiophen-7-yl}-propanoate from organic solvents, in particular from water, ethanol/water, methanol, acetonitrile/water or dichloromethane, more particularly from dichloromethane, in particular between 20 °C and 65 °C, more particularly ambient temperature, even more particularly at ca. 22 °C.

By anti-solvent crystallization: The Sodium salt amorphous form can be prepared by addition of a solution of sodium (S)-2-methoxy-3-{4-[2-(5-methyl-2-phenyl-oxazol-4-yl)-ethoxy]-benzo[b]thiophen-7-yl}-propanoate in organic solvents, in particular e.g. ethanol/water or dichloromethane, more particular in dichloromethane, in particular between 20 °C and 65 °C, more particularly ambient temperature, even more particularly at ca. 22 °C, to an anti-solvent (e.g. heptane).

The amorphous form of the sodium salt can be characterized by the lack of sharp X-ray diffraction peaks in its XRPD pattern.

The amorphous form of the sodium salt can also be characterized by an infrared spectrum having sharp bands at: 3391, 3061, 1599, 1555, 1508, 1413, 1348, 1268, 1235, 1179, 1143, 1101, 1046, 1024, 949, 932, 797, 774, 715 and 692 cm-1 (± 2 cm-1).

The amorphous form of the sodium salt can also be characterized by a Raman spectrum having sharp bands at 3072, 2926, 1643, 1608, 1572, 1556, 1510, 1451, 1387, 1350, 1233, 1174, 1025, 1003, 951, 845, 683, 617 and 444 cm-1 (± 3 cm-1).

The amorphous form of the sodium salt can also be characterized by a glass transition temperature (DSC) in the range of approximately 62 °C to 66 °C (the glass transition temperature is largely dependant on the water/solvent content).

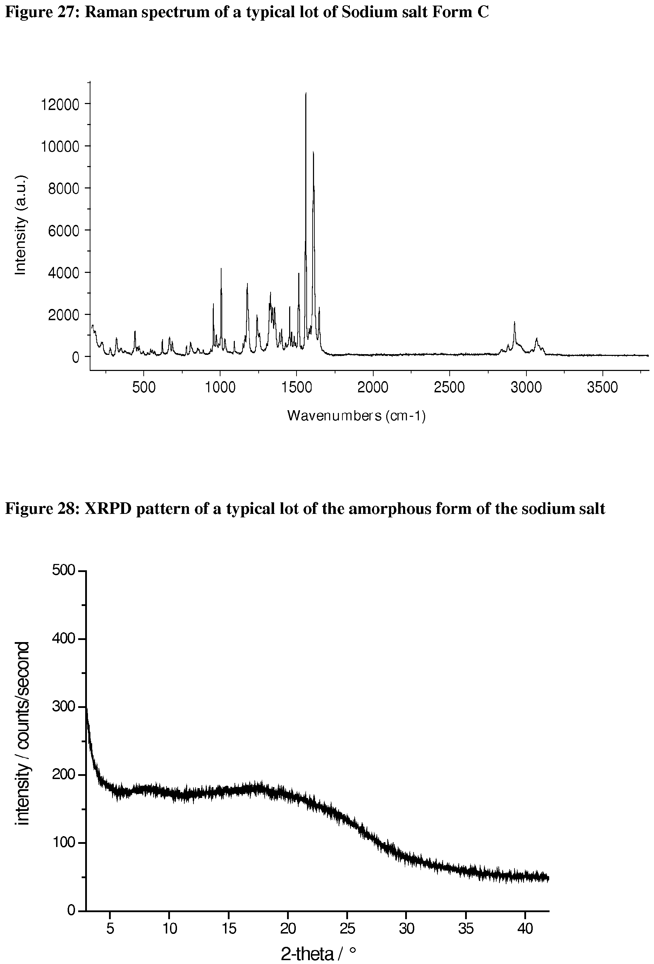

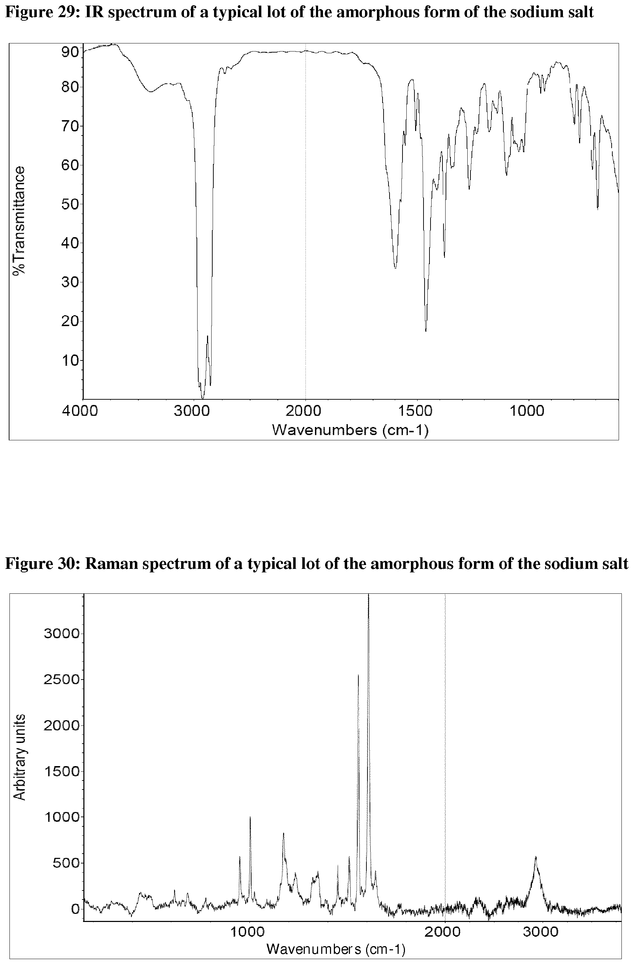

These characteristics and others are shown on figures 28 to 30.

Diisopropylammonium salt Form A is a slightly hygroscopic polymorphic form of diisopropylammonium (S)-2-methoxy-3-{4-[2-(5-methyl-2-phenyl-oxazol-4-yl)-ethoxy]-benzo[b]thiophen-7-yl}-propanoate with a decomposition temperature of approx. 100 °C.

Form A of Diisopropylammoinum (S)-2-methoxy-3-{4-[2-(5-methyl-2-phenyl-oxazol-4-yl)-ethoxy]-benzo[b]thiophen-7-yl}-propanoate can be prepared according to the following procedure.

By long-term slurry equilibration: Diisopropylammonium salt Form A can be prepared by long-term equilibration of Free acid Form A in diisopropylamine in particular at ambient temperature, in particular for several days, in particular at least two days.

Diisopropylammonium salt Form A can be characterized by an X-ray powder diffraction pattern obtained with a Cu Kα radiation having characteristic peaks expressed in degrees 2Theta at approximately: 6.3, 7.9, 10.2, 11.9, 12.7, 13.3, 13.8, 17.6, 18.6, 22.1, 23.6, 24.0, 24.4, 26.8, 27.1 and 29.5. The term "approximately" means in this context that there is an uncertainty in the measurements of the degrees 2Theta of ± 0.2 (expressed in degrees 2Theta).

Diisopropylammonium salt Form A can also be characterized by an infrared spectrum having sharp bands at: 3508, 3457, 3072, 3024, 2789, 2723, 2587, 2491, 2452, 1641, 1627, 1576, 1555, 1486, 1453, 1447, 1385, 1354, 1338, 1298, 1270, 1240, 1204, 1186, 1173, 1152, 1143, 1106, 1086, 1059, 1026, 977, 951, 936, 804, 798, 783, 717, 697, 669, and 626 cm-1 (± 2 cm-1).

Diisopropylammonium salt Form A can also be characterized by a Raman spectrum having sharp bands at: 3071, 2984, 2922, 2869, 1644, 1607, 1576, 1556, 1510, 1454, 1399, 1385, 1355, 1337, 1325, 1299, 1251, 1230, 1172, 1083, 1029, 1003, 970, 952, 805, 681, 619, 520, 436, 369, 336, 316, and 271 cm-1 (± 3 cm-1).

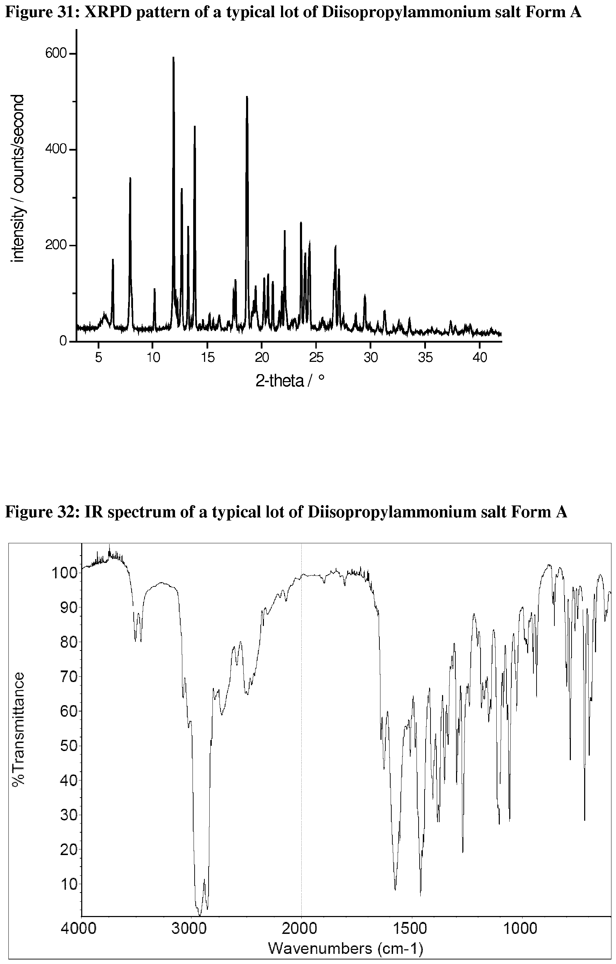

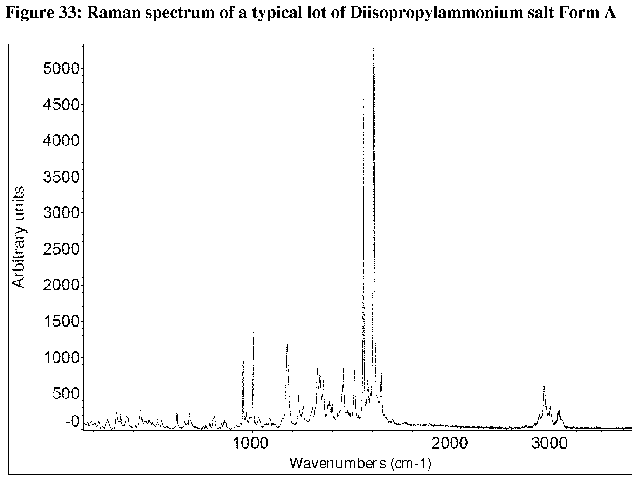

These characteristics and others are shown on figures 31 to 33.

The invention relates also to the following:
A pharmaceutical composition comprising a solid form according to the invention;

A pharmaceutical composition according to the invention for the prevention or the treatment of type II diabetes, acute coronary syndrome, insulin resistance, diabetic nephropathy or diabetic retinopathy;

A solid form according to the invention for use as a medicament; and

A solid form according the invention for the prevention or the treatment of type II diabetes, acute coronary syndrome, insulin resistance, diabetic nephropathy or diabetic retinopathy.
Figure 1 shows a XRPD pattern of a typical lot of form A of (S)-2-methoxy-3-{4-[2-(5-methyl-2-phenyl-oxazol-4-yl)-ethoxy]-benzo[b]thiophen-7-yl}-propionic acid.
Figure 2 shows an IR spectrum of a typical lot of form A of (S)-2-methoxy-3-{4-[2-(5-methyl-2-phenyl-oxazol-4-yl)-ethoxy]-benzo[b]thiophen-7-yl}-propionic acid.
Figure 3 shows a Raman spectrum of a typical lot of form A of (S)-2-methoxy-3-{4-[2-(5-methyl-2-phenyl-oxazol-4-yl)-ethoxy]-benzo[b]thiophen-7-yl}-propionic acid.
Figure 4 shows a XRPD pattern of a typical lot of form B of (S)-2-methoxy-3-{4-[2-(5-methyl-2-phenyl-oxazol-4-yl)-ethoxy]-benzo[b]thiophen-7-yl}-propionic acid.
Figure 5 shows an IR spectrum of a typical lot of form B of (S)-2-methoxy-3-{4-[2-(5-methyl-2-phenyl-oxazol-4-yl)-ethoxy]-benzo[b]thiophen-7-yl}-propionic acid.
Figure 6 shows a Raman spectrum of a typical lot of form B of (S)-2-methoxy-3-{4-[2-(5-methyl-2-phenyl-oxazol-4-yl)-ethoxy]-benzo[b]thiophen-7-yl}-propionic acid.
Figure 7 shows a XRPD pattern of a typical lot of form C of (S)-2-methoxy-3-{4-[2-(5-methyl-2-phenyl-oxazol-4-yl)-ethoxy]-benzo[b]thiophen-7-yl}-propionic acid.
Figure 8 shows an IR spectrum of a typical lot of form C of (S)-2-methoxy-3-{4-[2-(5-methyl-2-phenyl-oxazol-4-yl)-ethoxy]-benzo[b]thiophen-7-yl}-propionic acid.
Figure 9 shows a Raman spectrum of a typical lot of form C of (S)-2-methoxy-3-{4-[2-(5-methyl-2-phenyl-oxazol-4-yl)-ethoxy]-benzo[b]thiophen-7-yl}-propionic acid.
Figure 10 shows a XRPD pattern of a typical lot of form D of (S)-2-methoxy-3-{4-[2-(5-methyl-2-phenyl-oxazol-4-yl)-ethoxy]-benzo[b]thiophen-7-yl}-propionic acid.
Figure 11 shows an IR spectrum of a typical lot of form D of (S)-2-methoxy-3-{4-[2-(5-methyl-2-phenyl-oxazol-4-yl)-ethoxy]-benzo[b]thiophen-7-yl}-propionic acid.
Figure 12 shows a Raman spectrum of a typical lot of form D of (S)-2-methoxy-3-{4-[2-(5-methyl-2-phenyl-oxazol-4-yl)-ethoxy]-benzo[b]thiophen-7-yl}-propionic acid.
Figure 13 shows a XRPD pattern of a typical lot of form E of (S)-2-methoxy-3-{4-[2-(5-methyl-2-phenyl-oxazol-4-yl)-ethoxy]-benzo[b]thiophen-7-yl}-propionic acid.
Figure 14 shows an IR spectrum of a typical lot of form E of (S)-2-methoxy-3-{4-[2-(5-methyl-2-phenyl-oxazol-4-yl)-ethoxy]-benzo[b]thiophen-7-yl}-propionic acid.
Figure 15 shows a Raman spectrum of a typical lot of form E of (S)-2-methoxy-3-{4-[2-(5-methyl-2-phenyl-oxazol-4-yl)-ethoxy]-benzo[b]thiophen-7-yl}-propionic acid.
Figure 16 shows a XRPD pattern of a typical lot of the amorphous form of (S)-2-methoxy-3-{4-[2-(5-methyl-2-phenyl-oxazol-4-yl)-ethoxy]-benzo[b]thiophen-7-yl}-propionic acid.
Figure 17 shows an IR spectrum of a typical lot of the amorphous form of (S)-2-methoxy-3-{4-[2-(5-methyl-2-phenyl-oxazol-4-yl)-ethoxy]-benzo[b]thiophen-7-yl}-propionic acid.
Figure 18 shows a Raman spectrum of a typical lot of the amorphous form of (S)-2-methoxy-3-{4-[2-(5-methyl-2-phenyl-oxazol-4-yl)-ethoxy]-benzo[b]thiophen-7-yl}-propionic acid.
Figure 19 shows a XRPD pattern of a typical lot of form A of sodium (S)-2-methoxy-3-{4-[2-(5-methyl-2-phenyl-oxazol-4-yl)-ethoxy]-benzo[b]thiophen-7-yl}-propanoate.
Figure 20 shows an IR spectrum of a typical lot of form A of sodium (S)-2-methoxy-3-{4-[2-(5-methyl-2-phenyl-oxazol-4-yl)-ethoxy]-benzo[b]thiophen-7-yl}-propanoate.
Figure 21 shows a Raman spectrum of a typical lot of form A of sodium (S)-2-methoxy-3-{4-[2-(5-methyl-2-phenyl-oxazol-4-yl)-ethoxy]-benzo[b]thiophen-7-yl}-propanoate.
Figure 22 shows a XRPD pattern of a typical lot of form B of sodium (S)-2-methoxy-3-{4-[2-(5-methyl-2-phenyl-oxazol-4-yl)-ethoxy]-benzo[b]thiophen-7-yl}-propanoate.
Figure 23 shows an IR spectrum of a typical lot of form B of sodium (S)-2-methoxy-3-{4-[2-(5-methyl-2-phenyl-oxazol-4-yl)-ethoxy]-benzo[b]thiophen-7-yl}-propanoate.
Figure 24 shows a Raman spectrum of a typical lot of form B of sodium (S)-2-methoxy-3-{4-[2-(5-methyl-2-phenyl-oxazol-4-yl)-ethoxy]-benzo[b]thiophen-7-yl}-propanoate.
Figure 25 shows a XRPD pattern of a typical lot of form C of sodium (S)-2-methoxy-3-{4-[2-(5-methyl-2-phenyl-oxazol-4-yl)-ethoxy]-benzo[b]thiophen-7-yl}-propanoate.
Figure 26 shows an IR spectrum of a typical lot of form C of sodium (S)-2-methoxy-3-{4-[2-(5-methyl-2-phenyl-oxazol-4-yl)-ethoxy]-benzo[b]thiophen-7-yl}-propanoate.
Figure 27 shows a Raman spectrum of a typical lot of form C of sodium (S)-2-methoxy-3-{4-[2-(5-methyl-2-phenyl-oxazol-4-yl)-ethoxy]-benzo[b]thiophen-7-yl}-propanoate.
Figure 28 shows a XRPD pattern of a typical lot of the amorphous form of sodium (S)-2-methoxy-3-{4-[2-(5-methyl-2-phenyl-oxazol-4-yl)-ethoxy]-benzo[b]thiophen-7-yl}-propanoate.
Figure 29 shows an IR spectrum of a typical lot of the amorphous form of sodium (S)-2-methoxy-3-{4-[2-(5-methyl-2-phenyl-oxazol-4-yl)-ethoxy]-benzo[b]thiophen-7-yl}-propanoate.
Figure 30 shows a Raman spectrum of a typical lot of the amorphous form of sodium (S)-2-methoxy-3-{4-[2-(5-methyl-2-phenyl-oxazol-4-yl)-ethoxy]-benzo[b]thiophen-7-yl}-propanoate.
Figure 31 shows a XRPD pattern of a typical lot of form A of diisopropylammonium (S)-2-methoxy-3-{4-[2-(5-methyl-2-phenyl-oxazol-4-yl)-ethoxy]-benzo[b]thiophen-7-yl}-propanoate.
Figure 32 shows an IR spectrum of a typical lot of form A of diisopropylammonium (S)-2-methoxy-3-{4-[2-(5-methyl-2-phenyl-oxazol-4-yl)-ethoxy]-benzo[b]thiophen-7-yl}-propanoate.
Figure 33 shows a Raman spectrum of a typical lot of form A of diisopropylammonium (S)-2-methoxy-3-{4-[2-(5-methyl-2-phenyl-oxazol-4-yl)-ethoxy]-benzo[b]thiophen-7-yl}-propanoate.

The invention thus relates to a solid form as defined in Figure 19.

The invention is further illustrated by the following examples which have no limiting character.

### Examples

### Comparative Example 1

### Preparation of form A of (S)-2-methoxy-3-{4-[2-(5-methyl-2-phenyl-oxazol-4-yl)-ethoxy]-benzo[b]thiophen-7-yl}-propionic acid

15.5 g of crude (S)-2-methoxy-3-{4-[2-(5-methyl-2-phenyl-oxazol-4-yl)-ethoxy]-benzo[b]thiophen-7-yl}-propionic acid were dissolved in 300 mL of isopropyl acetate at reflux temperature (89 °C). The solution was cooled to 75 °C and agitated for 1 h. The resulting suspension was cooled from 75 to -10 °C within 2 h. After agitation at -10 °C for 30 minutes, the crystals were isolated by filtration, rinsed with 113 mL of cold isopropyl acetate and dried at 80 °C/12 mbar for 16 hours, yielding 13.8 g (89%) of colorless needles.

### Comparative Example 2

### Preparation of form B of (S)-2-methoxy-3-{4-[2-(5-methyl-2-phenyl-oxazol-4-yl)-ethoxy]-benzo[b]thiophen-7-yl}-propionic acid

427 mg of (S)-2-methoxy-3-{4-[2-(5-methyl-2-phenyl-oxazol-4-yl)-ethoxy]-benzo[b]thiophen-7-yl}-propionic acid in Form A were incubated at 120 °C in an open glass tube for 26 h. Then, tube was closed and rapidly cooled to ambient temperature. The powder was analyzed without further processing.

### Comparative Example 3

### Preparation of form C of (S)-2-methoxy-3-{4-[2-(5-methyl-2-phenyl-oxazol-4-yl)-ethoxy]-benzo[b]thiophen-7-yl}-propionic acid

284.3 mg of (S)-2-methoxy-3-{4-[2-(5-methyl-2-phenyl-oxazol-4-yl)-ethoxy]-benzo[b]thiophen-7-yl}-propionic acid were dissolved at 65 °C in 6.0 mL of trifluoroethanol. The clear solution was linearly cooled from 65 to 5 °C within 8 hours. The crystals were isolated by filtration and dried at 25 °C/<5 mbar for 6 days.

### Comparative Example 4

### Preparation of form D of (S)-2-methoxy-3-{4-[2-(5-methyl-2-phenyl-oxazol-4-yl)-ethoxy]-benzo[b]thiophen-7-yl}-propionic acid

611 mg of (S)-2-methoxy-3-{4-[2-(5-methyl-2-phenyl-oxazol-4-yl)-ethoxy]-benzo[b]thiophen-7-yl}-propionic acid were dissolved in a closed glass vial in 3.0 mL of chloroform at 60 °C. The clear solution was linearly cooled from 60 to 10 °C within 8 hours. 8 hours to 10 °C. Excess liquid was filtered off, and the residue was handled in wet stage.

### Comparative Example 5

### Preparation of form E of (S)-2-methoxy-3-{4-[2-(5-methyl-2-phenyl-oxazol-4-yl)-ethoxy]-benzo[b]thiophen-7-yl}-propionic acid

526 mg of (S)-2-methoxy-3-{4-[2-(5-methyl-2-phenyl-oxazol-4-yl)-ethoxy]-benzo[b]thiophen-7-yl}-propionic acid were dissolved in a closed glass vial in 4 mL of dichloromethane at 60 °C. The solution was linearly cooed from 60 to 25 °C within 8 hours. Excess liquid was filtered off, and the residue was handled in wet stage.

### Comparative Example 6

### Preparation of the amorphous form of (S)-2-methoxy-3-{4-[2-(5-methyl-2-phenyl-oxazol-4-yl)-ethoxy]-benzo[b]thiophen-7-yl}-propionic acid

215.9 mg of (S)-2-methoxy-3-{4-[2-(5-methyl-2-phenyl-oxazol-4-yl)-ethoxy]-benzo[b]thiophen-7-yl}-propionic acid were heated to 180 °C in an open glass tube. After obtaining a complete melt the tube was closed and submerged into cold water. After 10 min. the glass tube was removed and put into a desiccator at ambient temperature. The powder was analyzed without further processing.

### Example 7

### Preparation of form A of sodium (S)-2-methoxy-3-{4-[2-(5-methyl-2-phenyl-oxazol-4-yl)-ethoxy]-benzo[b]thiophen-7-yl}-propanoate

562.5 mg of (mainly amorphous) sodium (S)-2-methoxy-3-{4-[2-(5-methyl-2-phenyl-oxazol-4-yl)-ethoxy]-benzo[b]thiophen-7-yl}-propanoate were suspended in 6 mL of ethanol/water (5 %-v/v water) at 65 °C for 17 d. The product was isolated by filtration and dried at 25 °C/<5 mbar for 24 h.

### Comparative Example 8

### Preparation of form B of sodium (S)-2-methoxy-3-{4-[2-(5-methyl-2-phenyl-oxazol-4-yl)-ethoxy]-benzo[b]thiophen-7-yl}-propanoate

221.6 mg of sodium (S)-2-methoxy-3-{4-[2-(5-methyl-2-phenyl-oxazol-4-yl)-ethoxy]-benzo[b]thiophen-7-yl}-propanoate (Sodium salt Form A) were incubated in a glass tube at 225 °C under argon atmosphere for 15 minutes. After passive cooling to ambient temperature the powder was analyzed without further processing.

### Comparative Example 9

### Preparation of form C of sodium salt (S)-2-methoxy-3-{4-[2-(5-methyl-2-phenyl-oxazol-4-yl)-ethoxy]-benzo[b]thiophen-7-yl}-propanoate

112.2 mg of the amorphous form of sodium (S)-2-methoxy-3-{4-[2-(5-methyl-2-phenyl-oxazol-4-yl)-ethoxy]-benzo[b]thiophen-7-yl}-propanoate were incubated at 100 %-RH at ambient temperature for 11 days, and then handled in wet stage.

### Comparative Example 10

### Preparation of the amorphous form of sodium (S)-2-methoxy-3-{4-[2-(5-methyl-2-phenyl-oxazol-4-yl)-ethoxy]-benzo[b]thiophen-7-yl}-propanoate

198.3 mg of sodium (S)-2-methoxy-3-{4-[2-(5-methyl-2-phenyl-oxazol-4-yl)-ethoxy]-benzo[b]thiophen-7-yl}-propanoate were dissolved in 10 mL of deionized water. After polishing filtration into a 250 mL round bottom flask, the solution was flash frozen with liquid nitrogen. The frozen solution was freeze-dried (Christ ALPHA 2-4 apparatus) within 48 h. The colorless fluffy powder was analyzed without further processing.

### Comparative Example 11

### Preparation of form A of diisopropylammonium (S)-2-methoxy-3-{4-[2-(5-methyl-2-phenyl-oxazol-4-yl)-ethoxy]-benzo[b]thiophen-7-yl}-propanoate

476.5 mg of (S)-2-methoxy-3-{4-[2-(5-methyl-2-phenyl-oxazol-4-yl)-ethoxy]-benzo[b]thiophen-7-yl}-propionic acid were suspended in 3 mL of diisopropylamine at 22 °C for 6 d. The product was isolated by filtration and dried at 25 °C/<5 mbar for 24 h.

### Example 12

### Competing slurry experiment between Free acid Forms A, B and C

Description of a typical competing slurry experiment:
200 and 300 mg of form A of (S)-2-methoxy-3-{4-[2-(5-methyl-2-phenyl-oxazol-4-yl)-ethoxy]-benzo[b]thiophen-7-yl}-propionic acid were each suspended in ca. 1 mL of isopropyl acetate at 20 and 60 °C, respectively. After 24 h the two suspensions were seeded with approximately 10 mg of Free acid Form B and Free acid Form C. After 14 days the crystals were harvested by filtration and analyzed in wet stage by XRPD. Result: pure Free acid Form A is obtained.

## Claims

1. A solid form of sodium (S)-2-methoxy-3-{4-[2-(5-methyl-2-phenyl-oxazol-4-yl)-ethoxy]-benzo[b]thiophen-7-yl}-propanoate **characterized by** an X-ray powder diffraction pattern obtained with a Cu Kα radiation having characteristic peaks expressed in degrees 2Theta at approximately 4.7, 4.8, 5.3, 7.9, 8.8, 13.1, 13.5, 13.8, 14.1, 14.5, 17.6, 19.3, 19.9, 21.6, 23.1 and 23.4.

2. A solid form according to claim 1 **characterized by** an X-ray powder diffraction pattern obtained with a Cu Kα as shown in Figure 19.

3. A pharmaceutical composition comprising a solid form according to claim 1 or 2.

4. A pharmaceutical composition according to claim 3 for use in the prevention or the treatment of type II diabetes, acute coronary syndrome, insulin resistance, diabetic nephropathy or diabetic retinopathy.

5. A solid form according to claim 1 or 2 for use as a medicament.

6. A solid form according to claim 1 or 2 for use in the prevention or the treatment of type II diabetes, acute coronary syndrome, insulin resistance, diabetic nephropathy or diabetic retinopathy.

7. A process for the manufacture of a solid form according to claim 1 or 2, comprising the slurry equilibration of the partially or completely amorphous form of sodium (S)-2-methoxy-3-{4-[2-(5-methyl-2-phenyl-oxazol-4-yl)-ethoxy]-benzo[b]thiophen-7-yl}-propanoate in organic solvents between 20 °C and 80 °C.

8. A process according to claim 7, wherein the organic solvent is ethanol/water.

9. A process according to claim 7 or 8, wherein the slurry equilibration is between 60 °C and 70 °C.

10. A process for the manufacture of a solid form according to claim 1 or 2, comprising the incubation of the partially or completely amorphous form of sodium (S)-2-methoxy-3-{4-[2-(5-methyl-2-phenyl-oxazol-4-yl)-ethoxy]-benzo[b]thiophen-7-yl}-propanoate between 115 °C and 200 °C.

11. A process according to claim 10, wherein the incubation is between 170 °C and 190 °C.

12. A process for the manufacture of a solid form according to claim 1 or 2, comprising the cooling crystallization of sodium (S)-2-methoxy-3-{4-[2-(5-methyl-2-phenyl-oxazol-4-yl)-ethoxy]-benzo[b]thiophen-7-yl}-propanoate from organic solvents from a temperature between 50 and 70 °C to a temperature between 0 °C and 20 °C.

13. A process according to claim 12, wherein the organic solvent is ethanol/water.

## Patentansprüche

1. Feststoffform von Natrium-(S)-2-methoxy-3-{4-[2-(5-methyl-2-phenyl-oxazoI-4-yl)-ethoxy]-benzo[b]thiophen-7-yI}-propanoat, **gekennzeichnet durch** ein mit einer Cu-Kα-Strahlung erhaltenes Röntgenpulverdiffraktogramm mit in Grad 2Theta ausgedrückten charakteristischen Peaks bei ungefähr 4,7, 4,8, 5,3, 7,9, 8,8, 13,1, 13,5, 13,8, 14,1, 14,5, 17,6, 19,3, 19,9, 21,6, 23,1 und 23,4.

2. Feststoffform nach Anspruch 1, **gekennzeichnet durch** ein mit Cu-Kα erhaltenes Röntgenpulverdiffraktogramm wie in Fig. 19 gezeigt.

3. Pharmazeutische Zusammensetzung, umfassend eine Feststoffform nach den Ansprüchen 1 oder 2.

4. Pharmazeutische Zusammensetzung nach Anspruch 3 zur Verwendung bei der Prävention oder Behandlung von Typ-II-Diabetes, akutem Koronarsyndrom, Insulinresistenz, diabetischer Nephropathie oder diabetischer Retinopathie.

5. Feststoffform nach Anspruch 1 oder 2 zur Verwendung als Medikament.

6. Feststoffform nach Anspruch 1 oder 2 zur Verwendung bei der Prävention oder Behandlung von Typ-II-Diabetes, akutem Koronarsyndrom, Insulinresistenz, diabetischer Nephropathie oder diabetischer Retinopathie.

7. Verfahren zur Herstellung einer Feststoffform nach Anspruch 1 oder 2, umfassend die Aufschlämmungsäquilibrierung der teilweise oder vollständig amorphen Form von Natrium-(S)-2-methoxy-3-{4-[2-(5-methyl-2-phenyl-oxazol-4-yl)-ethoxy]-benzo[b]thiophen-7-yl}-propanoat in organischen Lösungsmitteln zwischen 20 °C und 80 °C.

8. Verfahren nach Anspruch 7, wobei das organische Lösungsmittel Ethanol/Wasser ist.

9. Verfahren nach Anspruch 7 oder 8, wobei die Aufschlämmungsäquilibrierung zwischen 60 °C und 70 °C erfolgt.

10. Verfahren zur Herstellung einer Feststoffform nach Anspruch 1 oder 2, umfassend die Inkubation der teilweise oder vollständig amorphen Form von Natrium-(S)-2-methoxy-3-{4-[2-(5-methyl-2-phenyl-oxazol-4-yl)-ethoxy]-benzo[b]thiophen-7-yl}-propanoat zwischen 115 °C und 200 °C.

11. Verfahren nach Anspruch 10, wobei die Inkubation zwischen 170 °C und 190 °C erfolgt.

12. Verfahren zur Herstellung einer Feststoffform nach Anspruch 1 oder 2, umfassend die Kühlungskristallisation von Natrium-(S)-2-methoxy-3-{4-[2-(5-methyl-2-phenyl-oxazol-4-yl)-ethoxy]-benzo[b]thiophen-7-yl}-propanoat aus organischen Lösungsmitteln bei einer Temperatur von zwischen 50 und 70 °C bis zu einer Temperatur von zwischen 0 °C und 20 °C.

13. Verfahren nach Anspruch 12, wobei das organische Lösungsmittel Ethanol/Wasser ist.

## Revendications

1. Forme solide du (S)-2-méthoxy-3-{4-[2-(5-méthyl-2-phényl-oxazoI-4-yl)-éthoxy]-benzo[b]thiophén-7-yI}-propanoate de sodium **caractérisée par** un diagramme de diffraction des rayons X sur poudre obtenu avec un rayonnement Cu Kα ayant des pics caractéristiques exprimés en degrés 2Thêta à environ 4,7, 4,8, 5,3, 7,9, 8,8, 13,1, 13,5, 13,8, 14,1, 14,5, 17,6, 19,3, 19,9, 21,6, 23,1 et 23,4.

2. Forme solide selon la revendication 1, **caractérisée par** un diagramme de diffraction des rayons X sur poudre obtenu avec un Cu Kα tel qu'illustré sur la figure 19.

3. Composition pharmaceutique comprenant une forme solide selon la revendication 1 ou 2.

4. Composition pharmaceutique selon la revendication 3 destinée à être utilisée dans la prévention ou le traitement du diabète de type II, du syndrome coronarien aigu, de l'insulinorésistance, de la néphropathie diabétique ou de la rétinopathie diabétique.

5. Forme solide selon la revendication 1 ou 2 destinée à être utilisée en tant que médicament.

6. Forme solide selon la revendication 1 ou 2 destinée à être utilisée dans la prévention ou le traitement du diabète de type II, du syndrome coronarien aigu, de l'insulinorésistance, de la néphropathie diabétique ou de la rétinopathie diabétique.

7. Procédé de production d'une forme solide selon la revendication 1 ou 2, comprenant l'équilibrage de la suspension de la forme partiellement ou totalement amorphe de (S)-2-méthoxy-3-{4-[2-(5-méthyl-2-phényl-oxazoI-4-yl)-éthoxy]-benzo[b]thiophén-7-yI}-propanoate de sodium dans des solvants organiques entre 20 °C et 80 °C.

8. Procédé selon la revendication 7, dans lequel le solvant organique est de l'éthanol/eau.

9. Procédé selon la revendication 7 ou 8, dans lequel l'équilibrage de la suspension est effectué entre 60 °C et 70 °C.

10. Procédé de production d'une forme solide selon la revendication 1 ou 2, comprenant l'incubation de la forme partiellement ou totalement amorphe de (S)-2-méthoxy-3-{4-[2-(5-méthyl-2-phényl-oxazoI-4-yl)-éthoxy]-benzo[b]thiophén-7-yI}-propanoate de sodium entre 115 °C et 200 °C.

11. Procédé selon la revendication 10, dans lequel l'incubation est effectuée entre 170 °C et 190 °C.

12. Procédé de production d'une forme solide selon la revendication 1 ou 2, comprenant la cristallisation par refroidissement du (S)-2-méthoxy-3-{4-[2-(5-méthyl-2-phényl-oxazoI-4-yl)-éthoxy]-benzo[b]thiophén-7-yI}-propanoate de sodium à partir de solvants organiques allant d'une température comprise entre 50 °C et 70 °C à une température comprise entre 0 °C et 20 °C.

13. Procédé selon la revendication 12, dans lequel le solvant organique est de l'éthanol/eau.
